# EUROPEAN PATENT SPECIFICATION

(11) **EP 3 714 701 B1**
(45) Date of publication and mention of the grant of the patent: **10.08.2022**
(21) Application number: 19791557.2
(22) Date of filing: 04.04.2019
(51) Int. Cl.: A61K 35/747, A61K 31/715, A61P 37/08, A23L 33/135

(54) **COMPOSITION FOR TYPE IV ALLERGY**
ZUSAMMENSETZUNG FÜR TYP-IV-ALLERGIE
COMPOSITION POUR ALLERGIE DE TYPE IV

(30) Priority: 25.04.2018 JP 2018084049
(43) Date of publication of application: 30.09.2020
(73) Proprietor: Sone Farm Co., Ltd., Tokyo 160-0022 (JP)
(72) Inventor: SUGIYAMA,Masanori, Hiroshima-shi, Hiroshima 732-0063 (JP)
(74) Representative: Papula Oy
(86) International application number: PCT/JP2019/014998
(87) International publication number: WO 2019/208151

(56) References cited:
- WO-A1-2012/133827
- WO-A1-2018/225556
- JP-A- 2005 137 357
- JP-A- 2010 138 147
- JP-A- 2011 142 907
- JP-A- 2017 014 231
- DATABASE WPI Week 201618 Thomson Scientific, London, GB; AN 2016-066872 XP002801541, & KR 2016 0008060 A (KOREA FOOD RES INST) 21 January 2016 (2016-01-21)
- J. BENYACOUB ET AL: "Immune modulation property of Lactobacillus paracasei NCC2461 (ST11) strain and impact on skin defences", BENEFICIAL MICROBES, vol. 5, no. 2, 2014, pages 129-136, XP055338869, NL ISSN: 1876-2883, DOI: 10.3920/BM2013.0014
- MAKINO S et al: "Development of Functional Yogurt Utilizing Lactobacillus for Producing Immunmodulation Polysaccharides (non-official translation)", Kagaku to Seibutsu, vol. 53, no. 10 , pages 709-714,
- VIDAL KARINE et al.: "Effect of Lactobacillus paracasei NCC2461 on Antigen-Specific T- Cell Mediated Immune Responses in Aged Mice", REJUVENATION RESEARCH, vol. 11, no. 5, 2008, pages 957-964, XP055647021, ISSN: 1549-1684, DOI: 10.1089/rej.2008.0780
- KOU XIAOHONG et al.: "A tolerant lactic acid bacteria, Lactobacillus paracasei, and its immunoregulatory function", Can. J. Microbiol, vol. 60, no. 11, 2014, pages 729-736, XP009521230, DOI: 10.1139/cjm-2014-0383
- NODA MASAFUMI et al.: "A novel structure of exopolysaccharide produced by a plant-derived lactic acid bacterium Lactobacillus paracasei IJH-SONE68", J. Biochem, vol. 164, no. 2, May 2018 (2018-05), pages 87-92, XP055561348, DOI: 10.1093/jb/mvy048
- NODA Masafumi et al: "Functionality Analysis of Inflammation from and Allergies to extracellular Polysaccharid Lactobacillus paracasei IJH-SONE68", Japanese journal of lactic Acid Bacteria, vol. 29, no. 2, 2 July 2018 (2018-07-02), page 117,

## Description

### Technical Field

The present invention relates to a composition for type IV allergy. More specifically, the present invention relates to a composition for use in the prevention or improvement of type IV allergy which is contact dermatitis, comprising, as an active ingredient, a cell body of a lactic acid bacterium belonging to *Lactobacillus paracasei,* a cultured product or fermented product thereof, or a polysaccharide produced thereby.

### Background Art

In recent years, it has been pointed out that allergies including hay fever, atopic dermatitis, and contact dermatitis are remarkably increased due to various causes such as changes of diet, deterioration of air pollution, allergens, and exposure to metallic decorations. Allergies are regarded as a systemic or local failure of a living body based on an immune response. Allergies are broadly classified into type I, II, and III allergies based on a humoral immune reaction caused by blood antibodies, and type IV allergy based on a cell-mediated immune reaction caused by sensitized lymphocytes. Type IV allergy is also called delayed allergy, cellular immunity, or tuberculin type, and is thought to be caused by sensitized T cells that specifically react with an antigen. It is thought that in type IV allergy, various physiologically active substances such as factors that activate macrophages are released from sensitized T cells that have reacted with an antigen, causing damage to surrounding tissues. Type IV allergy includes tuberculin reaction and contact dermatitis, and an intradermal reaction of this type of allergy usually appears as redness or induration 24 to 48 hours after intradermal injection of an antigen.

Drugs used for such allergies include antihistamines and steroids. It has, however, been pointed out that these drugs show various side effects, and they cannot be deemed to be always safe.

Under these circumstances, in recent years, lactic acid bacteria that are involved in an immune reaction and have an antiallergic action have attracted attention as a highly safe antiallergic material. For example, proposals have been made on lactic acid bacteria, such as *Lactobacillus brevis,* that suppress the production of IgE antibodies (Patent Document 1), a lactic acid bacterium of *Lactobacillus reuteri* (strain AD0002) having an effect of suppressing histamine release (Patent Document 2), a lactic acid bacterium of *Enterococcus faecium* that activates an intestinal immunity or type I helper T cells (Patent Documents 3 and 4), a lactic acid bacterium of *Lactobacillus paracasei* strain KW3110 having a Th1 immunity enhancing action and a Th2 immunosuppressive action (Non-Patent Document 1), and the like.

### Prior Art Documents

### Patent Documents

Patent Document 1: JPH 09-2959 A
Patent Document 2: JP 2000-95697 A
Patent Document 3: JP 2006-67881 A
Patent Document 4: JP 2006-104107 A

### Non-Patent Documents

Non-Patent Document 1: International archives of allergy and immunology 2004; 135;205-215
Database WPI, Week 201618, Thomson Scientific London, GB: AN 2016-066872 & KR 2016 0008060 (Korea Food Res Inst) describes a composition comprising *Lactobacillus paracasei.*
WO 2012/133827 discloses a novel lactic acid bacterium, and a pharmaceutical, food or drink, and feed containing the lactic acid bacterium.
JP 2011 142907 describes *Lactobacillus paracasei* strain LT12 having immunoregulatory activity.
JP 2005 137357 describes an antiallergic composition.

### Summary of Invention

### Problem to be solved by invention

Under such background arts, it has been desired to develop a new composition for anti-allergies containing a lactic acid bacterium as an active ingredient. The problem to be solved by the present invention is thus to provide a new composition for type IV allergy, particularly effective in the prevention or improvement of contact dermatitis, comprising a lactic acid bacterium as an active ingredient.

### Means for solving the problem

The present inventor has conducted intensive studies with the aim of developing a new composition for type IV allergy, particularly for the prevention or improvement of contact dermatitis. As a result, the present inventor has found that a lactic acid bacterium belonging to *Lactobacillus paracasei* has an action of suppressing mouse contact dermatitis model and an action of preventing or improving type IV allergy, which is contact dermatitis. On the basis of these findings, the present inventor has further studied and completed the present invention.

In one aspect of the present invention, the present invention relates to a composition for use in the prevention or improvement of type IV allergy, comprising, as an active ingredient, a cell body of a lactic acid bacterium belonging to *Lactobacillus paracasei,* a cultured product or fermented product thereof, or a polysaccharide produced thereby, as defined in the appended claims.

In the composition of the present invention, a cultured product or fermented product of the lactic acid bacterium is preferably the one that can be obtained by cultivating or fermenting the lactic acid bacteria in the presence of a fruit juice of pineapple genus plant.

In the composition of the present invention, a polysaccharide produced by the lactic acid bacterium includes a neutral polysaccharide having a structure in which N-acetylglucosamines are linked with each other via α-1,6 bond, and an acidic polysaccharide composed mainly of glucoses and mannoses.

The composition of the present invention is preferably a food or drink composition, and the food or drink includes a beverage, a functional food, a fermented food, and a supplement.

In addition, the composition of the present invention is preferably a pharmaceutical composition.

Furthermore, the composition of the present invention is preferably a feed composition or a cosmetic composition.

### Effect of Invention

The composition of the present invention has an action of suppressing mouse contact dermatitis model, is effective in the prevention or improvement of type IV allergy which is contact dermatitis. The composition of the present invention can be used as a food or drink, a medicine, a feed, and a cosmetic product, for the prevention or improvement of type IV allergy which is contact dermatitis. The composition of the present invention comprises, as an active ingredient, a cell body of a lactic acid bacterium belonging to *Lactobacillus paracasei,* a cultured product or fermented product thereof, or a polysaccharide produced thereby, as defined in the appended claims. Therefore, the composition of the present invention has high safety, can be applied for a long period of time, can be supplied in a large amount at low cost, and has extremely high utility and practicality.

### Brief Description of Drawings

[Fig. 1] Fig. 1 shows microscope photographs of *Lactobacillus paracasei* strain IJH-SONE68. (A) in Fig. 1 is a gram-stained microscope photograph, and (B) in Fig. 1 is a scanning electron microscope (SEM) photograph.
[Fig. 2] Fig. 2 illustrates an isolation profile of anion exchange chromatography (TOYOPEARL DEAE-650M resin (Tosoh Corporation)) of exopolysaccharides from *Lactobacillus paracasei* strain IJH-SONE68. The exopolysaccharides were eluted with NaCl having a gradient concentration of 0 mM to 500 mM (broken line), and the exopolysaccharides in each fraction were monitored at 490 nm by a phenol sulfuric acid method (straight line).
[Fig. 3] Fig. 3 illustrates each NMR profile obtained by subjecting a neutral exopolysaccharide, which was obtained by purifying exopolysaccharides from *Lactobacillus paracasei* strain IJH-SONE68 with anion exchange column chromatography, to proton-NMR and carbon-NMR. (A) in Fig. 3 is the NMR profile of proton-NMR, and (B) in Fig. 3 is the NMR profile of carbon-NMR.
[Fig. 4] Fig. 4 illustrates results of structurally analyzing a neutral exopolysaccharide on the basis of the NMR profiles. These structural analysis results revealed that the neutral exopolysaccharide of *Lactobacillus paracasei* strain IJH-SONE68 has a structure in which N-acetylglucosamines are linked with each other via α-1,6 bond.
[Fig. 5] Fig. 5 is a graph (Turkey test) illustrating that a cell body or polysaccharide of *Lactobacillus paracasei* strain IJH-SONE68, when orally administered to mouse contact dermatitis models, had an action of suppressing the contact dermatitis.
[Fig. 6] Fig. 6 is a graph (t test according to Welch's method) illustrating that a cell body or polysaccharide of *Lactobacillus paracasei* strain IJH-SONE68, when orally administered to mouse contact dermatitis models, had an action of suppressing the contact dermatitis.
[Fig. 7] Fig. 7 is a graph (Turkey test) illustrating that a cell body or polysaccharide of *Lactobacillus paracasei* strain IJH-SONE68, when applied on mouse contact dermatitis models, had an action of suppressing the contact dermatitis.
[Fig. 8] Fig. 8 is a graph (t test according to Welch's method) illustrating that a cell body or polysaccharide of *Lactobacillus paracasei* strain IJH-SONE68, when applied on mouse contact dermatitis models, had an action of suppressing the contact dermatitis.
[Fig. 9] Fig. 9 is a graph illustrating that a pineapple juice fermented with *Lactobacillus paracasei* strain IJH-SONE68, when orally administered to mouse contact dermatitis models, had an action of improving the contact dermatitis.
[Fig. 10] Fig. 10 is a graph illustrating that neutral and acidic polysaccharides produced by *Lactobacillus paracasei* strain IJH-SONE68, when orally administered to mouse contact dermatitis models, had an action of improving the contact dermatitis.
[Fig. 11] Fig. 11 is a graph illustrating that neutral and acidic polysaccharides produced by *Lactobacillus paracasei* strain IJH-SONE68, when orally administered to mouse contact dermatitis models, had an action of suppressing the expression of inflammatory cytokine IL-4 in the mouse contact dermatitis models.
[Fig. 12] Fig. 12 is a graph illustrating that neutral and acidic polysaccharides produced by *Lactobacillus paracasei* strain IJH-SONE68, when orally administered to mouse contact dermatitis models, had an action of suppressing an increase in serum IgE level in the mouse contact dermatitis models.
[Fig. 13] Fig. 13 is a photograph illustrating results of observing with microscope appearances of auricles of mouse contact dermatitis models, to which neutral and acidic polysaccharides produced by *Lactobacillus paracasei* strain IJH-SONE68 have orally been administered.

### Embodiments for Carrying out Invention

The following detailed disclosures are made on the composition provided by the present invention for the prevention of improvement of type IV allergy, comprising, as an active ingredient, a cell body of a lactic acid bacterium belonging to *Lactobacillus paracasei,* a cultured product or fermented product thereof, or a polysaccharide produced thereby, as defined in the appended claims.

### 1. The lactic acid bacterium in the present invention

The lactic acid bacterium in the present invention is a lactic acid bacterium belonging to *Lactobacillus paracasei* and preferably a lactic acid bacterium derived from a fig. Specifically, the lactic acid bacterium includes *Lactobacillus paracasei* strain IJH-SONE68 that was isolated and identified from leaves of a fig according to the present invention. This strain was nationally deposited under the accession number of NITE P-02242 at Patent Microorganisms Depositary, National Institute of Technology and Evaluation (#122, 2-5-8 Kazusakamatari, Kisarazu-shi, Chiba 292-0818, Japan) on April 19, 2016. The deposition was then transferred to an international deposit under the Budapest Treaty and given the international deposit accession number of NITE BP-02242 on May 26, 2017.

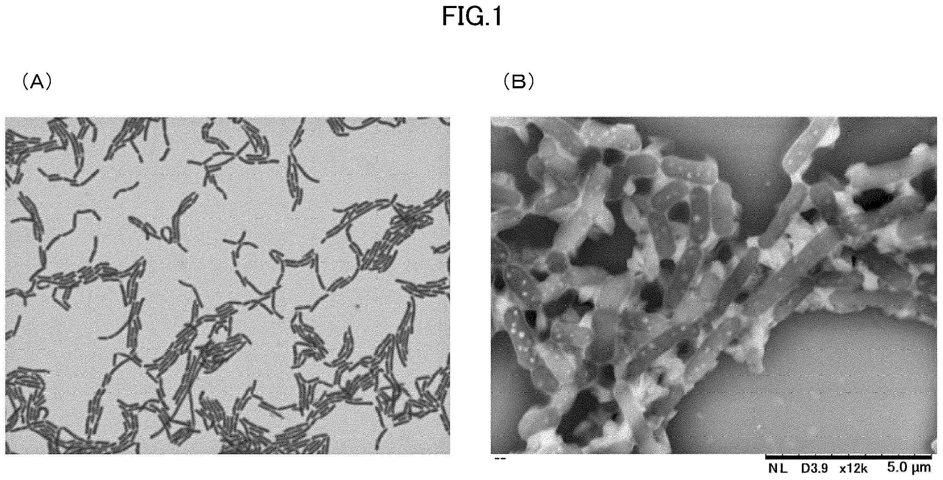

As illustrated in the photograph of Fig. 1, *Lactobacillus paracasei* strain IJH-SONE68 is a catalase-negative, gram-positive bacillus, and has mycological properties of forming a white colony and the mycological characteristic of conditional heterolactic fermentation. Furthermore, the strain has an ability to produce polysaccharides.

### 2. The active ingredient in the present invention

The composition of the present invention contains, as an active ingredient, a cell body of the above-mentioned lactic acid bacterium, a cultured product or fermented product thereof, or a polysaccharide produced thereby.

The lactic acid bacterium can be cultured by a commonly used culture method such as liquid stationary culture, using a commonly used MRS medium or a modified medium thereof. The lactic acid bacterium can promote its growth by the culture in the presence of a fruit juice of a pineapple genus plant or its extract (WO 2011/046194 A). In addition, the lactic acid bacterium can also promote its growth by the culture in the presence of sake lees, sake lees extract or sake lees enzymes (JPH 03-172171 A, JPH 05-15366 A, and JP 2835548 B). As the lactic acid bacterium, a culture obtained after the cultivation may be used as it is, the obtained culture solution may be diluted or concentrated to be used, or the cell body recovered from the culture may be used. In addition, as long as the effect of the present invention is not impaired, various additional operations such as heat and freeze-dry may also be performed after the cultivation. The cell body of the lactic acid bacterium may be a viable or dead cell body, in which polysaccharides are adhered to the cell surface of the lactic acid bacterium, and may include both the viable and dead cell bodies. The dead cell body may be crushed and preferably has polysaccharides adhered to the cell surface thereof. In addition, the fermented product of the lactic acid bacterium can be obtained by fermenting the lactic acid bacterium usually using glucose or the like as a nutrient source, and further using yeast extract, fruit juice of pineapple genus plants, sake lees, distilled spirit lees, etc., if necessary.

Polysaccharides produced by the lactic acid bacterium can be obtained by isolating and purifying them from a culture of lactic acid bacteria belonging to *Lactobacillus paracasei* according to an ordinary method. Specifically, for example, the polysaccharides can be obtained by removing the cell body from a culture of lactic acid bacteria belonging to *Lactobacillus paracasei* by centrifugation, and precipitating polysaccharides from the obtained culture using ethanol, acetone, or the like. In addition, the polysaccharides can be obtained by further isolating and purifying them by ion exchange chromatography. The thus obtained polysaccharides produced by the lactic acid bacterium include neutral and acidic polysaccharides.

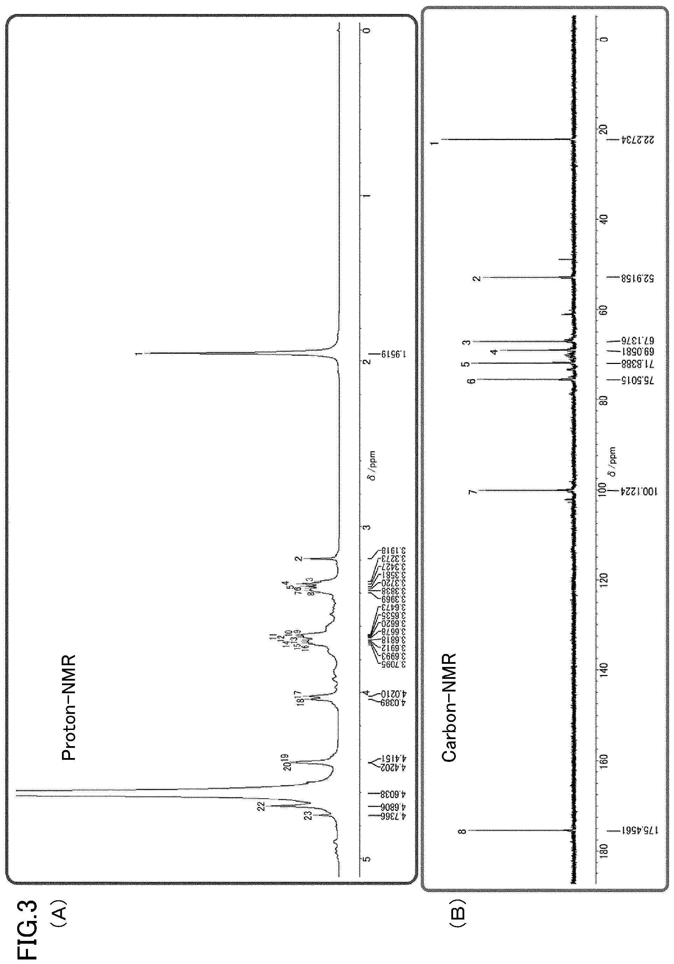

In the present invention, the polysaccharides produced by the lactic acid bacterium include a neutral polysaccharide having a structure in which N-acetylglucosamines are linked with each other via α-1,6 bond, and/or an acidic polysaccharide composed mainly of glucoses and mannoses. This neutral polysaccharide can be obtained by isolating and purifying polysaccharides obtained from a culture of *Lactobacillus paracasei* strain IJH-SONE68 by anion exchange chromatography, as disclosed in Example 2 herein below. It was revealed from the proton-NMR and carbon-NMR profiles illustrated in Fig. 3 that this neutral polysaccharide has a structure in which N-acetylglucosamines are linked with each other by α-1,6 bonds. In addition, the *Lactobacillus paracasei* strain IJH-SONE68 secretes an acidic polysaccharide mainly composed of glucose and mannose outside the cell body. More specifically, this acidic polysaccharide is composed of glucose, mannose, galactose, and rhamnose, and their composition ratio is approximately 10:170: 2:1.

### 3. The composition in the present invention

The composition of the present invention can be used in various forms of a food or drink composition, a pharmaceutical composition, a feed composition, and a cosmetic composition.

The food or drink of the food or drink composition are not particularly limited but include beverages such as soft drinks, carbonated drinks, nutritional drinks, fruit juice beverages, and lactic acid bacteria beverages, concentrated stock solutions of these beverages, powders for the preparation of these beverages, and the like; ice cream, sherbet and ice confectionery such as shaved ice; confectioneries such as candy, gummy, cereal, chewing gum, candy, gum, chocolate, tablet candy, snack, biscuit, jelly, jam, cream, and baked confectionery; dairy products such as processed milk, milk drink, fermented milk, drink yogurt, and butter; bread; enteral nutritious food, liquid food, childcare milk, sports drink; food such as puree; seat sake; and other functional foods. In addition, the food or drink may be supplements, and the supplements may be in the form of, for example, granules, powders, or tablets.

The food or drink as disclosed above may be prepared by adding a cell body of a lactic acid bacterium belonging to *Lactobacillus paracasei,* a cultured product or fermented product thereof, or a polysaccharide produced thereby, to raw materials of food or drink, or may be prepared in the same manner as an usual food or drink. The addition of a cell body of a lactic acid bacterium belonging to *Lactobacillus paracasei,* a cultured product or fermented product thereof, or a polysaccharide produced thereby may be performed at any stage of the process of preparing the food or drink. The food or drink may be prepared after a fermentation process of the added lactic acid bacteria. Examples of such food or drink include fermented foods such as lactic acid bacterium beverages and fermented milks.

The content of a cell body of the lactic acid bacterium or a cultured product or fermented product thereof in the food or drink composition may be appropriately determined depending on the embodiment of the food or drink, but is the one so that a cell body of the lactic acid bacterium is usually contained in the food or drink composition preferably in the range of 1 × 10⁶ to 1×10¹² cfu/g or 1 ×10⁶ to 1×10¹² cfu/ml, more preferably in the range of 1×10⁷ to 1×10¹¹ cfu/g or 1×10⁷ to 1×10¹¹ cfu/ml. In the case where the lactic acid bacterium is a dead cell body, the cfu/g or cfu/ml can be replaced with the number of cells per g or the number of cells per ml. In the case of the polysaccharide produced by the lactic acid bacterium, the polysaccharide is usually contained in the food or drink composition at an amount of 0.001% or more by weight, preferably at an amount of 0.01% or more by weight, in terms of the weight of the polysaccharide.

The pharmaceutical composition is usually used by blending a cell body of the lactic acid bacterium, a cultured product or fermented product thereof or a polysaccharide produced thereby with a physiologically acceptable liquid or solid of a pharmaceutical carrier usually used, followed by the formulation. The dosage form of the pharmaceutical composition is not particularly limited, but includes tablets, pills, powders, solutions, suspensions, emulsions, granules, capsules, syrups, suppositories, injections, ointments, patches, eye drops, and nose drops.

The content of a cell body of the lactic acid bacterium or a cultured product or fermented product thereof in the pharmaceutical composition may be appropriately determined depending on the dosage form, the dosage regimen, the age and sex of a subject, the kind of disease, the degree of disease, other conditions and the like, but is the one so that a cell body of the lactic acid bacterium is usually contained in the pharmaceutical composition preferably in the range of 1 × 10⁶ to 1×10¹² cfu/g or 1 ×10⁶ to 1×10¹² cfu/ml, more preferably in the range of 1×10⁷ to 1×10¹¹ cfu/g or 1×10⁷ to 1×10¹¹ cfu/ml. In the case where the lactic acid bacterium is a dead cell body, the cfu/g or cfu/ml can be replaced with the number of cells per g or the number of cells per ml. In the case of the polysaccharide produced by the lactic acid bacterium, the polysaccharide is usually contained in the pharmaceutical composition at an amount of 0.001% or more by weight, preferably at an amount of 0.01% or more by weight, in terms of the weight of the polysaccharide.

The administration timing of the pharmaceutical composition of the present invention is not particularly limited but may be appropriately chosen depending on a subject to be applied. The pharmaceutical composition may also be administered prophylactically or used in a maintenance therapy. The administration mode may be preferably appropriately determined depending on the dosage form, age, sex and other conditions of the administered subject, the degree of symptoms of the administered subject, and the like. In any case, the pharmaceutical composition of the present invention may be administered once per day, administered dividedly into two or more times or administered once every several days or weeks.

Examples of the feed of a feed composition include pet food, livestock feed and fish feed. Such a feed may be prepared by mixing common feed, for example, cereals, cakes, brans, fish meals, bone meals, oils and fats, skim milk powders, wheys, bitterns, mineral feeds, yeasts, or the like with a cell body of the lactic acid bacterium, a cultured product or fermented product thereof or a polysaccharide produced thereby. In addition, for example, like the case of silage, a feed may be prepared through a fermentation process with the lactic acid bacterium added thereto. The prepared feed may be orally administered to general mammals, livestock, farmed fishes, pet animals or the like. In the case of farmed fishes, it may be adopted to spread fermented products, to which a cell body of the lactic acid bacterium, a cultured product or fermented product thereof or a polysaccharide produced thereby is added, to the farmed place of fishes.

The content of a cell body of the lactic acid bacterium or a cultured product or fermented product thereof in the feed composition may be appropriately determined depending on the embodiment of the feed or the administered subject, but is the one so that a cell body of the lactic acid bacterium is usually contained in the feed composition preferably in the range of 1×10⁶ to 1×10¹² cfu/g or 1×10⁶ to 1×10¹² cfu/ml, more preferably in the range of 1×10⁷ to 1×10¹¹ cfu/g or 1 × 10⁷ to 1×10¹¹ cfu/ml. In the case where the lactic acid bacterium is a dead cell body, the cfu/g or cfu/ml can be replaced with the number of cells per g or the number of cells per ml. In the case of the polysaccharide produced by the lactic acid bacterium, the polysaccharide is usually contained in the feed composition at an amount of 0.001% or more by weight, preferably at an amount of 0.01% or more by weight, in terms of the weight of the polysaccharide.

Examples of the cosmetic product of the cosmetic composition containing the lactic acid bacterium of the present invention include washing agents such as soaps, body shampoos, cleansing creams, and facial cleansers; creams such as lotions, vanishing creams, cold creams, emollient creams, and massage creams; milky lotions and serums. The cosmetic composition of the present invention may be obtained by adding a cell body of the lactic acid bacterium of the present invention, a cultured product or fermented product thereof, or a polysaccharide produced thereby, to materials usually used in the above-mentioned cosmetic products.

In the cosmetic composition of the present invention, it is preferable to use, for example, a lactic acid-fermented egg white obtained by adding the lactic acid bacterium of the present invention to a liquid egg white prepared by breaking eggs of birds such as chickens and removing the egg yolk, followed by the fermentation. In general, such lactic acid fermentation is preferably performed by using glucose or the like as a nutrient source, adding a fermentation promoting substance such as yeast extract, if necessary, and fermenting them. The form of the lactic acid-fermented egg white may be, for example, liquid, powder, cream, paste or jelly, depending on the cosmetic product to be blended therewith.

The content of a cell body of the lactic acid bacterium or a cultured product or fermented product thereof in the cosmetic composition of the present invention may be appropriately determined depending on the embodiment of the cosmetic product or the applied subject, but is the one so that a cell body of the lactic acid bacterium is usually contained in the cosmetic composition preferably in the range of 1×10⁶ to 1×10¹² cfu/g or 1×10⁶ to 1×10¹² cfu/ml, more preferably in the range of 1×10⁷ to 1×10¹¹ cfu/g or 1 ×10⁷ to 1×10¹¹ cfu/ml. In the case where the lactic acid bacterium is a dead cell body, the cfu/g or cfu/ml can be replaced with the number of cells per g or the number of cells per ml. In the case of the lactic acid-fermented egg white, the content of the egg white is usually 0.001% or more by weight, preferably 0.01% or more by weight, in terms of the dried egg white. In the case of the polysaccharide produced by the lactic acid bacterium, the polysaccharide is usually contained in the cosmetic composition at an amount of 0.001% or more by weight, preferably at an amount of 0.01% or more by weight, in terms of the weight of the polysaccharide.

### 4. Use of the composition of the present invention

A cell body of a lactic acid bacterium belonging to *Lactobacillus paracasei,* a cultured product or fermented product thereof, or a polysaccharide produced thereby has an action of suppressing mouse contact dermatitis and an action of suppressing type IV allergy, which is contact dermatitis. In addition, a cell body of a lactic acid bacterium belonging to *Lactobacillus paracasei,* a cultured product or fermented product thereof, or a polysaccharide produced thereby suppresses the expression of inflammatory cytokine IL-4 and an increase in serum IgE level and can suppress an inflammatory symptom. Therefore, a cell body of a lactic acid bacterium belonging to *Lactobacillus paracasei,* a cultured product or fermented product thereof, or a polysaccharide produced thereby is for use in the prevention or improvement of type IV allergy which is contact dermatitis. In particular, a cell body of a lactic acid bacterium belonging to *Lactobacillus paracasei,* a cultured product or fermented product thereof, or a polysaccharide produced thereby has an action of suppressing contact dermatitis and is for use in the prevention or improvement of allergic contact dermatitis due to skin contact of drugs or the like. In addition, the composition of the present invention is effective in the prevention or improvement of type IV allergy and can be therefore used as materials for food or drink for the maintenance and enhancement of health.

### Examples

Hereinafter, the present invention will be disclosed in more detail with reference to examples, but the present invention is not limited by these examples.

### Example 1

### Isolation and identification of lactic acid bacterium

### 1. Isolation of lactic acid bacterium sample

The leaves, stems, and fruits of a fig (variety "TOYOMITSU HIME") were chosen and cut into pieces of 2 to 3 mm using sterilized tweezers and scissors. Every five to six pieces were each then placed in a sterilized test tube containing MRS liquid medium, and statically cultured at 28°C and 37°C until the MRS medium as a standard medium for a lactic acid bacterium became turbid (proliferated). By the way, it took 2 to 4 days for the proliferation of the lactic acid bacterium candidate strains to be visible.

A part of each culture liquid of the lactic acid bacterium candidate strains was subjected to a line drawing paint on MRS agar medium using a disposable loop, followed by stationary culture. Among colonies formed on the agar medium, all of differently colored, lustrous and shaped colonies were picked up and subjected to a line drawing paint on a fresh MRS agar medium, and the colonies were purified.

H₂O₂ test was performed for each purified colony to verify the presence or absence of the production of a catalase enzyme. This is a test method for observing the presence or absence of oxygen generated when catalase is present, which is observed when microbial bodies are exposed to 10% H₂O₂ solution. By the way, a lactic acid bacterium produces no catalase.

As a result of attempting the search and isolation from a fig, one lactic acid bacterium candidate strain showing catalase-negative was obtained from the leaves of a fig as the isolation source.

### 2. Identification of the isolated strain

The aforementioned lactic acid bacterium candidate strain was again cultured in MRS liquid medium, and the microbial bodies were obtained by centrifugation. After the microbial bodies were treated with cell wall lytic enzyme, a genomic DNA was extracted using DNAzol reagent.

According to the method as disclosed in Lane, DJ (1991), "16S/23S rRNA sequencing", Nucleic Acid Techniques in Bacterial Systematics, pp. 115-175, edited by E. Stackebrandt & M. Goodfellow. Chichester: Wiley, a genomic DNA PCR was performed using a genomic DNA as a template and using 27f primer and 1525r primer, thereby to amplify 16S rDNA part. Then, an objective fragment was recovered from agarose gel according to NucleoSpin Gel and PCR Clean-up kit (manufactured by Mahalay Nagel). A sequencing reaction by a dye terminator method for sequencing a base sequence was performed with Big Dye Terminator Cycle Sequencing FS Ready Reaction Kit ver. 3.1 (manufactured by ThermoFisher Scientific), and analysis was made with ABI PRISM 3130xl Genetic Analyzer (manufactured by ThermoFisher Scientific). The base sequence of the analyzed 16S rDNA was subjected to a homology search by BLAST program and compared with the database of DNA data bank (DDBJ/EMBL/GenBank) to make a taxonomic identification on the isolated strain.

The lactic acid bacterium candidate strain isolated from leaves of a fig was named strain IJH-SONE68 and identified as *Lactobacillus paracasei* because it was 100% identical to a base sequence which was in the strain of *Lactobacillus paracasei* R094 already registered in DNA data bank (DDBJ/EMBL/GenBank) and which had NR-025880 as the accession number of the base sequence.

This strain was nationally deposited under the accession number of NITE P-02242 at Patent Microorganisms Depositary, National Institute of Technology and Evaluation (#122, 2-5-8 Kazusakamatari, Kisarazu-shi, Chiba 292-0818, Japan) on April 19, 2016. The deposition was then transferred to an international deposit under the Budapest Treaty and given the international deposit accession number of NITE BP-02242 on May 26, 2017.

### 3. Mycological properties of separated and identified lactic acid bacterium

The aforementioned isolated and identified lactic acid bacterium strain IJH-SONE68 was a catalase-negative, gram-positive rod and had a white colony forming property, as shown in the photograph of Fig. 1, and further had the characteristic of conditional hetero-lactic acid fermentation and the ability of producing polysaccharides.

### 4. Saccharide assimilation ability of the isolated and identified lactic acid bacterium (1) Test method of assimilation ability

The strain IJH-SONE68 was investigated for the assimilation ability of 49 kinds of saccharides according to the following test method.

The strain IJH-SONE68 was statically cultured in MRS liquid medium until the proliferation stationary phase. The cell bodies obtained by centrifugation were washed with an appropriate amount of a suspension medium (manufactured by BioMeieux), and finally suspended in 2 mL of a suspension medium. A portion of the resultant suspension was added to 5 mL of a suspension medium to determine an amount (n) for McFarland turbidity to become 2. Subsequently, 2n of a microbial solution was added to API 50 CHL medium (manufactured by BioMerieux), and this solution was dispended to each well of API 50 CHL kit (manufactured by BioMerieux, 49 kinds of saccharides were coated on the bottom of each well). Finally, mineral oil was overlaid and set in a tray containing a sterilized water. After culturing at 37°C for 48 hours, the presence or absence of the assimilation ability was assessed by observing the change in color tone in each well.

### 5. Test results of the assimilation ability

Table 1 shows the results of investigating the assimilation ability of the strain IJH-SONE68 against 49 kinds of saccharides.

**[Table 1]**

| Assimilation abilities of the strain IJH-SONE68 against saccharides | |
|---|---|
| Substrates | Assimilation abilities |
| Control | - |
| Glycerol | - |
| Erythritol | - |
| D-Arabinose | - |
| L-Arabinose | - |
| D-Ribose | + |
| D-Xylose | - |
| L-Xylose | - |
| D-Adonitol | + |
| Methy 1-βD-xylopyranoside | - |
| D-Galactose | + |
| D-Glucose | + |
| D-Fructose | + |
| D-Mannose | + |
| L-Sorbose | + |
| L-Rhamnose | - |
| Dulcitol | - |
| Inositol | - |
| D-Mannitol | + |
| D-Sorbitol | - |
| Methy 1-αD-mannopyranoside | - |
| Methy 1-αD-glucopyranoside | - |
| N-Acetylglucosamine | + |
| Amygdalin | - |
| Arbutin | - |
| Esculin + Ferric citrate | + |
| Salicin | + |
| D-Cellobiose | + |
| D-Maltose | + |
| D-Lactose | - |
| D-Melibiose | - |
| D-Sucrose | + |
| D - Trehalose | + |
| Inulin | - |
| D-Melezitose | + |
| D-Raffinose | - |
| Starch | - |
| Glycogen | - |
| Xylitol | - |
| Gentibiose | + |
| D-Turranose | - |
| D-Lyxose | - |
| D-Tagatose | + |
| D-Fucose | - |
| L-Fucose | - |
| D-Arabitol | - |
| L-Arabitol | - |
| Gluconic acid | + |
| 2-Ketogluconic acid | - |
| 5-Ketogluconic acid | - |

| | |
|---|---|
| In Table 1, + indicates the possession of assimilation ability, and - indicates no possession of assimilation ability. | |

### Example 2

### 1. Isolation and purification of exopolysaccharides produced by the strain IJH-SONE68

Exopolysaccharides produced by the strain IJH-SONE68 were isolated and purified according to the following method.

The strain IJH-SONE68 was statically cultured in MRS liquid medium until the proliferation stationary phase. 5 mL of the resultant culture solution was used as a seed culture solution, and inoculated on 5 L of a semisynthetic medium for producing exopolysaccharides (the composition thereof will be disclosed below), followed by static culture at 37°C for 120 hours. After the resultant culture solution was cooled to 4°C, proteins contained in the culture supernatant were denatured, and 202.5 mL of a 100% trichloroacetic acid aqueous solution was added thereto, mixed and allowed to stand for 30 minutes to remove them as precipitates in a later step. After the precipitates were removed by centrifugation, an equal amount of acetone was added to the collected supernatant and mixed, and the resultant mixture was allowed to stand at 4°C overnight to precipitate polysaccharides produced by the strain IJH-SONE68. The precipitates were collected by centrifugation, and the resultant precipitates were then washed with 250 mL of 70% ethanol. After the precipitates were air-dried, 75 mL of 50 mM Tris-HCl buffer (pH 8.0) was added to the resultant precipitates and mixed for 1 hour to dissolve the precipitates. After insoluble impurities were removed by centrifugation to recover a supernatant, 750 µL of 1 mg/mL DNase solution (Worthington, Inc.) and 750 µL of 1 mg/mL RNase solution (Nacalai Tesque, Inc.) were each added to the recovered supernatant, followed by being allowed to react at 37°C for 8 hours. Subsequently, 750 µL of 2 mg/mL proteinase K solution (manufactured by Wako Pure Chemical Industries, Ltd.) was added, and the resultant mixture was reacted at 37°C for 16 hours. The resultant solution after the reaction was cooled to 4°C, the added enzymes were each denatured, and 8.75 mL of a 100% trichloroacetic acid aqueous solution was then added thereto, mixed and allowed to stand for 1 hour to remove the enzymes as precipitates in the next centrifugation. The resultant precipitates were removed by centrifugation to obtain a supernatant, 262.5 mL of 100% ethanol was added to the obtained supernatant, the resultant mixture was thoroughly mixed, and the polysaccharides produced by the strain IJH-SONE68 strain were then recovered as precipitates by centrifugation. After the precipitates were washed with 50 mL of 70% ethanol, the precipitates were air-dried, an appropriate amount (about 25 mL) of a purified water was added thereto, and the resultant mixture was allowed to stand overnight at 4°C to dissolve the polysaccharides. For the polysaccharides sample after the dissolution, small molecules such as monosaccharides in the recovered sample were removed using an ultrafiltration unit (Merck Ltd.) of 10,000 MWCO while replacing the solvent with a purified water, and a purified polysaccharide sample was thus obtained.

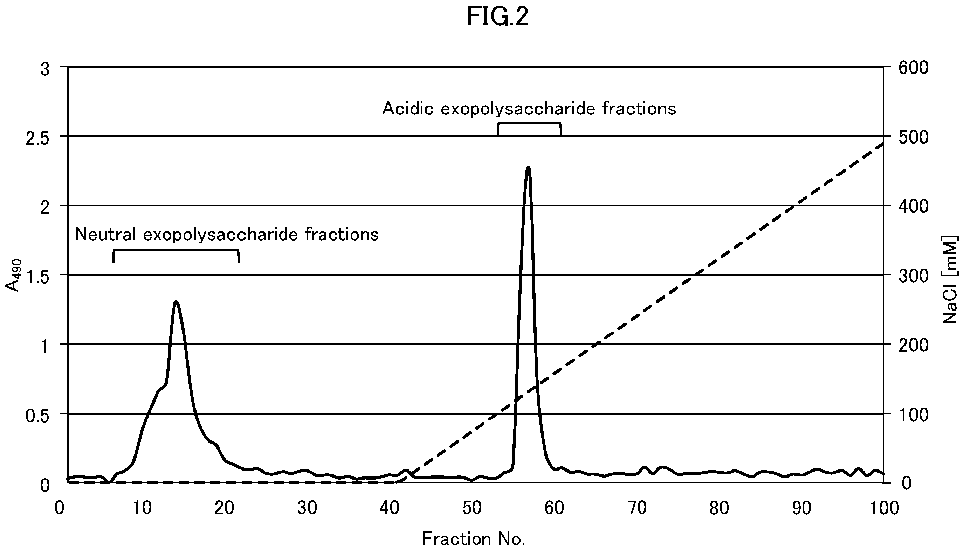

The purified polysaccharide sample was applied to an open column (2.5 × 22 cm) packed with TOYOPEARL DEAE-650M resin (Tosoh Corporation) previously equilibrated with 50 mM Tris-HCl buffer (pH 8.0), and column work was performed to isolate and purify the sample to neutral polysaccharide fractions and acidic polysaccharide fractions. The same buffer was used as an elution solution, and a flow rate was fixed at 1 mL/min. In addition, eluates were collected in different test tubes at every 6 mL. First, from the beginning to 240 minutes, elution was made with the same buffer (Test Tube Nos. 1 to 40). Next, from 240 minutes to 600 minutes, a concentration gradient of 0 to 500 mM NaCl was prepared using the same buffer, and elution was continued with the gradient (Test Tube Nos. 41 to 100). The column isolation spectrum is illustrated in Fig. 2. After the presence of polysaccharides was confirmed by a phenol sulfuric acid method (disclosed below) for all the samples eluted in the test tubes, the confirmed solutions in the test tubes were collected as neutral polysaccharide fractions and acidic polysaccharide fraction, respectively. For each fraction, an ultrafiltration unit of 10,000 MWCO was used to remove small molecules such as monosaccharides in the recovered sample while replacing the solvent with purified water.

As a result, neutral polysaccharide fractions and acidic polysaccharide fractions were isolated and purified as exopolysaccharides produced by the strain IJH-SONE68.

A semisynthetic medium for producing polysaccharides was prepared by modifying a medium disclosed in Kimmel SA, Roberts RF., "Development of a growth medium suitable for exopolysaccharide production by Lactobacillus delbrueckii ssp. Bulgaricus RR.", Int. J. Food Microbiol., 40, 87-92 (1998), as follows:
Semisynthetic medium for producing polysaccharides [g/L]

| | |
|---|---|
| Glucose | 20 |
| Tween 80 | 1.0 |
| Ammonium citrate | 2.0 |
| Sodium acetate | 5.0 |
| MgSO₄•7H₂O | 0.1 |
| MnSO₄•5H₂O | 0.05 |
| K₂HPO₄ | 2.0 |
| Bacto casitone | 10.0 |
| Vitamin Soln. | 2 mL |
| Trace element Soln. | 1 mL |

| | |
|---|---|
| Vitamin Soln. | [g/L] |
| 4-Aminobenzoic acid | 0.05 |
| Biotin | 0.001 |
| Folic acid | 0.025 |
| Lipoic acid | 0.025 |
| Nicotinic acid | 0.1 |
| Pantothenic acid | 0.05 |
| Pyridoxamin-HCl | 0.25 |
| Vitamin B₁₂ | 0.05 |
| Pyridoxine | 0.025 |
| Riboflavin | 0.05 |
| Thiamine | 0.1 |

Trace element Soln is disclosed in Kets EPW, Galinski EA, de Bont JAM Carnitine: "A novel compatible solute in Lactobacillus plantarum", Arch. Microbiol., 192, 243-248 (1994), and the composition is as follows:

| | |
|---|---|
| Trace element Soln. | [g/L] |
| 25% HCl | 10 mL |
| FeCl₂•4H₂O | 1.5 |
| CoCl₂•6H₂O | 0.19 |
| MnCl₂•4H₂O | 0.1 |
| ZnCl₂ | 0.07 |
| H₃BO₃ | 0.006 |
| Na₂MoO₄•2H₂O | 0.036 |
| NiCl₂•6H₂O | 0.024 |
| CuCl₂•2H₂O | 0.002 |

Phenol sulfuric acid method (DuBois M, Gilles KA, Hamilton JK, Rebers PA, Smith F., "Colorimetric method for determination of sugars and related substances", Anal. Chem., 28, 350-356 (1956))

30 µL of a subject sample was mixed with an equal amount of 5 w/v% phenol aqueous solution, and 150 µL of a concentrated sulfuric acid was added to the resultant mixture and mixed with each other to allow a reaction to start. Immediately after 10 minutes, the reaction solution was cooled by ice to stop the reaction. The concentration of saccharides was obtained by measuring the absorbance of the reaction solution at 490 nm. The concentration was determined using a calibration curve prepared by performing the same experiment using glucose as a standard.

### 2. Structural analysis of neutral exopolysaccharide

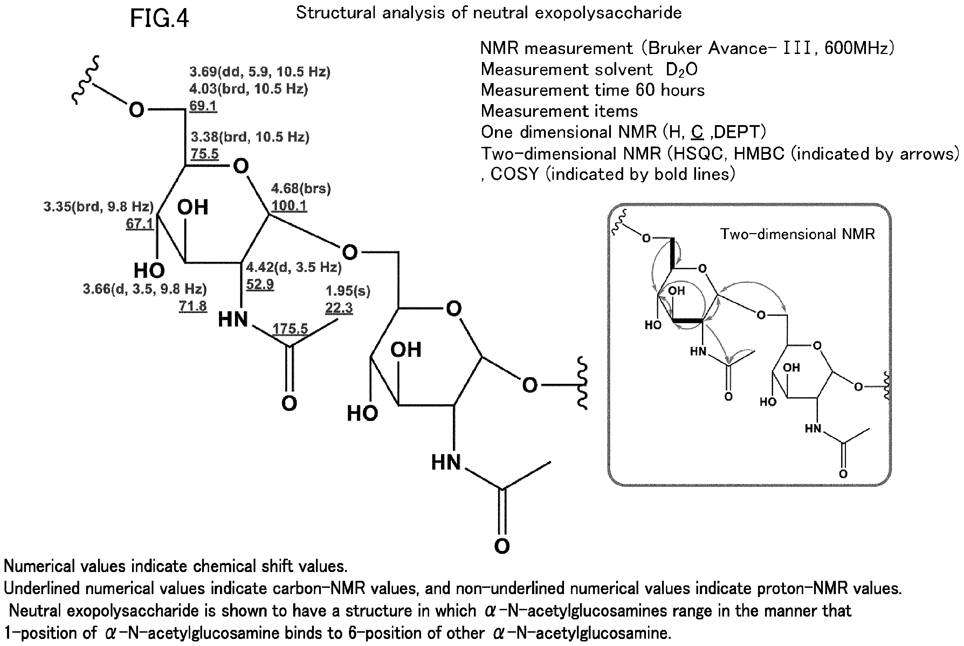

The neutral exopolysaccharide purified by the aforementioned anion exchange column chromatography (TOYOPEARL DEAE-650 M resin (Tosoh Corporation)) was subjected to proton-NMR and carbon-NMR, and the obtained NMR profiles are each illustrated in Fig. 3. The structural analysis results of the neutral exopolysaccharide from these NMR profiles are illustrated in Fig. 4.

From the structural analysis results, it was revealed that the neutral exopolysaccharide produced by the strain IJH-SONE68 has a structure in which N-acetylglucosamines are linked with each other via α-1,6 bond.

### 3. Saccharide composition analysis of acidic exopolysaccharide

The saccharide composition analysis of the aforementioned acidic exopolysaccharide purified by the anion exchange column chromatography was performed by measuring the composition by a high performance liquid chromatography (HPLC) method.

A 7-fold diluted sample solution was prepared by mixing 10 µL of the purified acidic exopolysaccharide (7.3 mg/mL) and 60 µL of water and placed in a test tube. 20 µL of the diluted sample solution was collected from the test tube, dried under reduced pressure, and 100 µL of 2 mol/L trifluoroacetic acid was added thereto to dissolve the dried sample. The resultant solution was substituted with nitrogen, sealed under a reduced pressure, hydrolyzed at 100°C for 6 hours, and then dried under a reduced pressure. To the obtained residue, 200 µL of water was added, dissolved, and filtrated with 0.22 µm filter, to obtain a sample solution for measurement. The sample solution for measurement was 10-fold diluted with water to obtain a sample solution for dilution measurement. 50 µL of each of these sample solutions was analyzed. HPLC system: LC-20A system (Shimadzu Corporation) and spectrofluorophotometer M-10AxL (Shimadzu Corporation) were used as analytical instruments. The analysis conditions were as follows:
Column: TSK-gel Sugar AXG 4.6 mml. D. × 15 cm (Tosoh Corporation)
Column temperature: 70°C
Mobile phase: 0.5 mol/L potassium borate buffer, pH 8.7
Mobile phase flow rate: 0.4 mL/min
Post column labeling: reaction reagent: 1 w/v% arginine •3 w/v% boric acid
Reaction reagent flow rate: 0.5 mL/min
Reaction temperature: 150°C
Detection wavelength: Ex. 320 nm, Em. 430 nm

Chromatograms of samples prepared from the acidic exopolysaccharide and calibration curve data of each monosaccharide were obtained. The concentrations of constituent saccharides of the acidic exopolysaccharide in the samples were determined from calibration curves. The obtained results are shown in Table 2.

**[Table 2]**

| Constituent saccharides of acidic exopolysaccharide | | |
|---|---|---|
| Acidic exopolysaccharide | | Concentration in sample (mg/mL) |
| Monosaccharides | Rhamnose | 0.0204 |
| | Ribose | n.d. |
| | Mannose | 3.43 |
| | Arabinose | n.d. |
| | Galactose | 0.0384 |
| | Xylose | n.d. |
| | Glucose | 0.219 |

| | | |
|---|---|---|
| In the Table, n.d. indicates no detection. | | |

### Example 3

### Suppression action on mouse contact dermatitis model by oral administration of IJH-SONE68 to the model

According to a method disclosed below, investigations were made on the suppression action of the cell body of the lactic acid bacterium of the strain IJH-SONE68 obtained in Example 1 and the exopolysaccharide produced by the strain IJH-SONE68 obtained in Example 2 on mouse contact dermatitis, when they were orally administered to the mouse contact dermatitis model.

### 1. Test method

### (1) Preparation of sample for mouse test

The strain IJH-SONE68 was cultured in MRS medium for 48 hours, cell bodies were collected from the resultant culture broth by centrifugation, the collected cell bodies were resuspended in a purified water, and the resultant suspension was used as a sample for mouse test (equal to about 1 ×10¹² cfu/mL). This suspension was used as a viable cell body sample, as it was. In addition, this suspension was subjected to heat sterilization treatment at 121°C for 20 minutes, and the obtained suspension was used as a dead cell body sample.

On the other hand, exopolysaccharides produced by the strain IJH-SONE68 were purified according to the method disclosed in Example 2 from the supernatant of a culture broth obtained by cultivating the strain in modified-SDM medium, and the purified exopolysaccharides (the mixture of the neutral polysaccharide fractions and the acidic polysaccharide fractions in Example 2) were supplied to an exopolysaccharide administration group (the concentration: 1 mg/mL).

### (2) Reagents used in test

Picryl chloride was purchased from Tokyo Chemical Industry Co., Ltd. to be used in test.

### (3) Method

The mice used were male BALB/cA Jcl mice (7-week old at the time of arrival, CLEA Japan, Inc.). Five mice were housed in a breeding cage and bred in an animal breeding room in which environment was adjusted to temperature of 20 to 26°C, humidity of 40 to 60%, and lighting time of 12 hours (8:00 to 20:00). The mice were allowed to freely ingest solid feed MF (Oriental Yeast Co., Ltd.). In addition, with regard to a drinking water, the mice were allowed to freely ingest a purified water.

After carried in the cage, the mice were acclimated for 7 days. After acclimated breeding, the mice were divided into groups as follows: Group A: a control group; Group B: a group in which suspension of viable cell bodies of the strain IJH-SONE68 was administered to the mice; Group C: a group in which suspension of dead cell bodies of the strain IJH-SONE68 was administered to the mice; and Group D: a group in which suspension of exopolysaccharides derived from the strain IJH-SONE68 was administered to the mice. That time point was designated as Day 0, and the sample was orally administered to the mice once per day from Day 0 for 14 consecutive days. The daily dose volume was 0.20 mL/mouse.

On Day 6, all mice were allowed to memorize an antigen information by applying a 7 w/v% picryl chloride solution on both hands and feet (20 µL), thorax and abdomen (100 µL), and back (100 µL). On Day 14, the thickness of right auricle of the mice was measured in advance with a caliper, and then an allergic reaction was induced by applying 20 µL of a 1 w/v% picryl chloride solution on the front and back of the right auricle. Twenty-four hours later (Day 15), the thickness of the auricle was measured, and the difference before and after the induction (Δ auricle thickness [mm]) was compared among the groups. Data were shown as mean ± standard error. Tukey's test or t-test by Welch's method was used for a significant difference test among the groups, and it was determined that there was a significant difference when a risk factor (p value) was less than 0.05.

The presence or absence of the suppression action of each sample on the mouse contact dermatitis model was evaluated according to the method disclosed above.

### 2. Test result

Table 3 shows Δ auricle thickness in each group.

**[Table 3]**

| Increased amount of auricle thickness in each mouse used in experiment | | | | |
|---|---|---|---|---|
| Mouse number | Group A | Group B | Group C | Group D |
| 1 | 0.16 | 0.14 | 0.12 | 0.12 |
| 2 | 0.12 | 0.12 | 0.08 | 0.12 |
| 3 | 0.14 | 0.12 | 0.10 | 0.10 |
| 4 | 0.12 | 0.10 | 0.12 | 0.10 |
| 5 | 0.18 | 0.10 | - | 0.10 |
| Average | 0.144 | 0.116 | 0.105 | 0.108 |
| SE | 0.012 | 0.007 | 0.010 | 0.005 |

| | | | | |
|---|---|---|---|---|
| Group A (control group), Group B (viable cell body administration group), Group C (dead cell body administration group), Group D (undivided EPS administration group) | | | | |

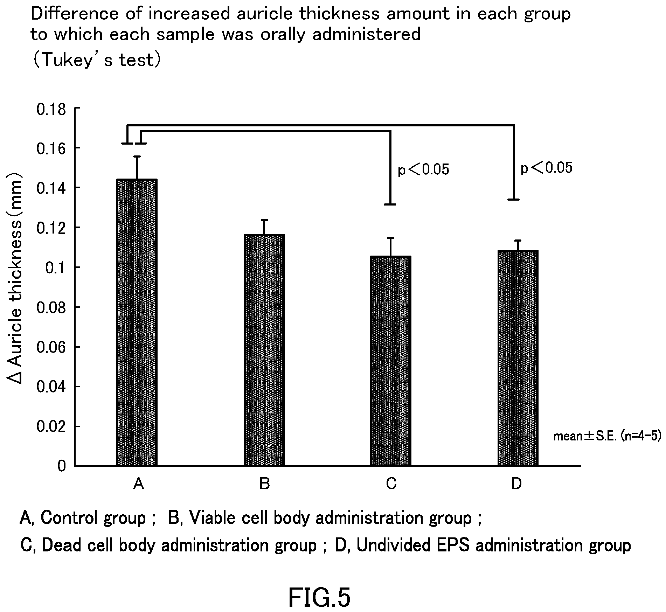

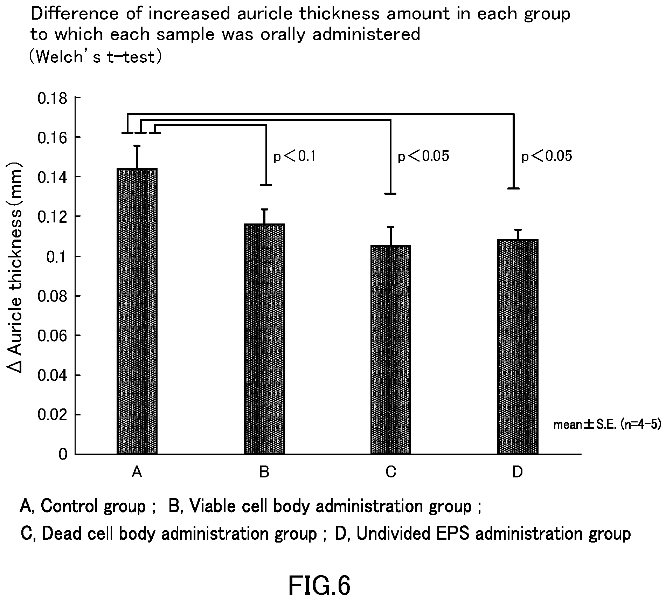

The results are also shown in Fig. 5 (Tukey test) and Fig. 6 (t test by Welch's method). It was confirmed from Fig. 5 that an increase in the auricle thickness due to inflammation was significantly suppressed (p < 0.05) in Groups C and D, as compared to the control group. It was also confirmed from Fig. 6 that when comparison was made only between the control group and Group B, an increase in the auricle thickness tended to be suppressed (p < 0.1).

It was shown from the above results that the oral administration of the strain IJH-SONE68 or the exopolysaccharides produced by the strain, when repeated before and after the antigen sensitization, improved the symptoms of the picryl chloride-induced contact dermatitis model.

### Example 4

### Suppression action on mouse contact dermatitis model by the application of IJH-SONE68 on the model

According to a method disclosed below, investigations were made on the suppression action of the exopolysaccharide obtained in Example 2, which was produced by the strain IJH-SONE68 obtained in Example 1, on mouse contact dermatitis, when the exopolysaccharide was applied on the mouse contact dermatitis model.

### 1. Test method

### (1) Preparation of sample for mouse test

From the supernatant of a culture broth obtained by cultivating the strain IJH-SONE68 in modified-SDM medium, exopolysaccharides produced by the strain were purified by the method disclosed in Example 2 to be supplied to experiments (1 mg/mL). The same concentration was used for a dipotassium glycyrrhizinate aqueous solution used as a control of an existing anti-inflammatory drug.

### (2) Reagents used in test

Picryl chloride was purchased from Tokyo Chemical Industry Co., Ltd. to be used in test.

### (3) Method

The mice used were male BALB/cA Jcl mice (7-week old at the time of arrival, CLEA Japan, Inc.). Five mice were housed in a breeding cage and bred in an animal breeding room in which environment was adjusted to temperature of 20 to 26°C, humidity of 40 to 60%, and lighting time of 12 hours (8:00 to 20:00). The mice were allowed to freely ingest solid feed MF (Oriental Yeast Co., Ltd.). In addition, with regard to a drinking water, the mice were allowed to freely ingest a purified water.

After carried in the cage, the mice were acclimated for 7 days. After acclimated breeding, the mice were divided into groups as follows: Group A: a control group; Group B: a group in which dipotassium glycyrrhizinate was applied on the mice; Group C: a group in which an undivided EPS (the mixture of the neutral polysaccharide fractions and the acidic polysaccharide fractions obtained in Example 2) was applied on the mice; Group D: a group in which a neutral EPS (the neutral polysaccharide fractions obtained in Example 2) was applied on the mice; and Group E: a group in which an acidic EPS (the acidic polysaccharide fractions obtained in Example 2) was applied on the mice. That time point was designated as Day 0, and all mice were allowed to memorize an antigen information by applying a 7 w/v% picryl chloride solution on both hands and feet (20 µL), and thorax and abdomen (150 µL).

On Day 6, the thickness of right auricle of the mice was measured in advance with a caliper, and then an allergic reaction was induced by applying 20 µL of a 1 w/v% picryl chloride solution on the front and back of the right auricle. Two hours after the application, 20 µL of each sample was applied on the back and front of the right auricle of the mice of each group. Eighteen hours later (Day 7), the thickness of the auricle was measured, and the difference before and after the induction (Δ auricle thickness [mm]) was compared among the groups. Data were shown as mean ± standard error. Tukey's test or t test by Welch's method was used for a significant difference test among the groups, and it was determined that there was a significant difference when a risk factor (p value) was less than 0.05.

The presence or absence of the suppression action of each sample on the mouse contact dermatitis model was evaluated according to the method disclosed above.

### 2. Test result

Table 4 shows Δ auricle thickness in each group.

**[Table 4]**

| Increased amount of auricle thickness in each mouse used in experiment | | | | | |
|---|---|---|---|---|---|
| Mouse number | Group A | Group B | Group C | Group D | Group E |
| 1 | 0.24 | 0.22 | 0.16 | 0.22 | 0.20 |
| 2 | 0.22 | 0.22 | 0.24 | 0.26 | 0.22 |
| 3 | 0.28 | 0.22 | 0.18 | 0.08 | 0.24 |
| 4 | 0.32 | 0.20 | 0.18 | 0.16 | 0.20 |
| 5 | 0.34 | 0.06 | 0.20 | 0.22 | 0.16 |
| Average | 0.28 | 0.184 | 0.192 | 0.188 | 0.204 |
| SE | 0.023 | 0.031 | 0.014 | 0.031 | 0.013 |

| | | | | | |
|---|---|---|---|---|---|
| Group A (control group), Group B (dipotassium glycyrrhizinate application group), Group C (undivided EPS application group), Group D (neutral EPS application group), Group E (acidic EPS application group) | | | | | |

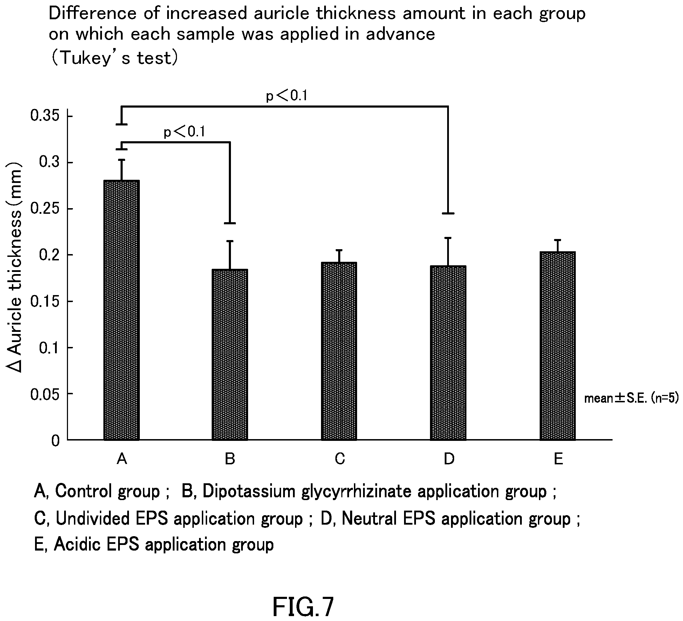

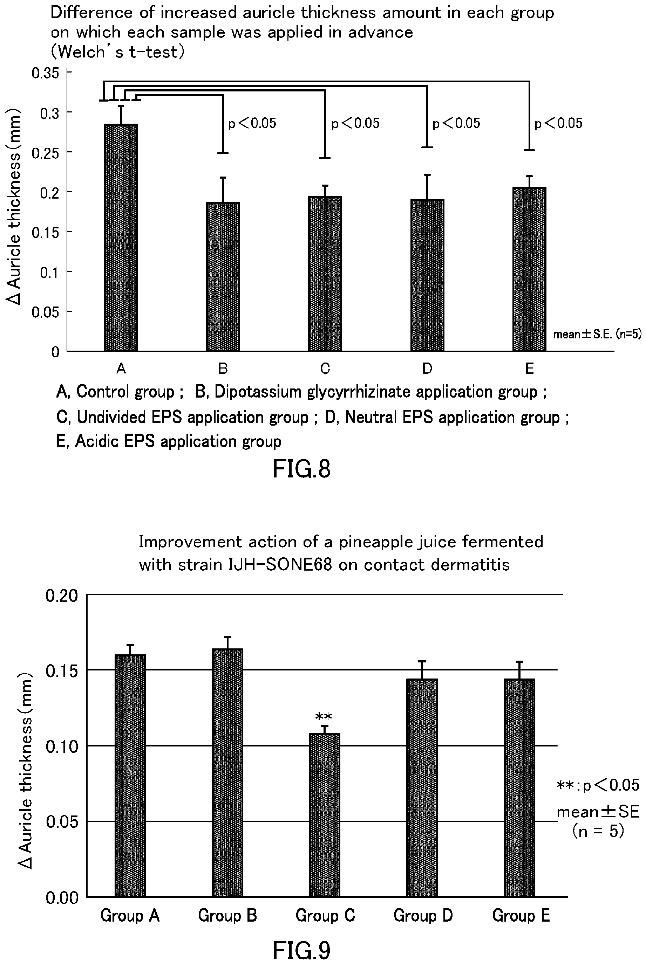

The results are also shown in Fig. 7 (Tukey test) and Fig. 8 (t test by Welch's method). It was confirmed from Fig. 7 that an increase in the auricle thickness due to inflammation tended to be suppressed (p < 0.1) in Groups B and D, as compared to the control group. It was also confirmed from Fig. 8 that when comparison was made between the control group and each group, an increase in the auricle thickness was significantly suppressed (p < 0.05).

It was shown from the above results that the application of the exopolysaccharides produced by the strain IJH-SONE68 on the inflammation site improved the symptoms of the picryl chloride-induced contact dermatitis model.

### Example 5

### Improvement action of IJH-SONE68-fermented solution on mouse contact dermatitis model

According to a method disclosed below, investigations were made on the improvement action of a pineapple juice fermented with the strain IJH-SONE68 obtained in Example 1 on mouse contact dermatitis model, when the juice was orally administered to the mouse contact dermatitis model.

### 1. Test method

The mice used were male BALB/cA Jcl mice (7-week old at the time of arrival, CLEA Japan, Inc.). Five mice were housed in a breeding cage and bred in an animal breeding room in which environment was adjusted to temperature of 20 to 26°C, humidity of 40 to 60%, and lighting time of 12 hours (8:00 to 20:00). The mice were allowed to freely ingest solid feed MF (Oriental Yeast Co., Ltd.). In addition, with regard to a drinking water, the mice were allowed to freely ingest a purified water. After carried in the cage, the mice were acclimated for 7 days. After acclimated breeding, the mice were divided into groups as follows:
Group A: a positive control group (a sterile distilled water administration group)
Group B: an unfermented pineapple juice administration group
Group C: a group in which an undiluted pineapple juice fermented with the strain IJH-SONE68 was administered to the mice
Group D: a group in which a 10-foled diluted pineapple juice fermented with the strain IJH-SONE68 was administered to the mice
Group E: a group in which a 100-fold diluted pineapple juice fermented with the strain IJH-SONE68 was administered to the mice

Here, the undiluted fermented pineapple juice refers to a fermented solution obtained by cultivating the strain IJH-SONE68 in 100% pineapple juice (containing 1 w/v% distillation residue of distilled spirits) for 48 hours. The 10-fold diluted juice refers to one obtained by 10-foled diluting the undiluted juice with a sterile distilled water, and the 100-fold diluted juice refers to one obtained by 100-foled diluting the undiluted juice with a sterile distilled water.

The time point at which the above grouping was performed was designated as Day 0, and each of the above samples was orally administered to the mice at a frequency of once per day from Day 0 for 14 consecutive days. The daily dose volume was 0.20 mL/mouse. On Day 6, all mice were allowed to memorize an antigen information by applying a 7 w/v% picryl chloride solution on both hands and feet (20 µL), thorax and abdomen (100 µL), and back (100 µL). On Day 13, the thickness of right auricle of the mice was measured in advance with a caliper, and then an allergic reaction was induced by applying 20 µL of a 1 w/v% picryl chloride solution on the front and back of the right auricle. Twenty-four hours later (Day 14), the thickness of the auricle was measured, and the difference before and after the induction (Δ auricle thickness [mm]) was compared among the groups. Data were shown as mean ± standard error. Tukey-Kramer method was used for a significant difference test among the groups, and it was determined that there was a significant difference when a risk factor (p value) was less than 0.05.

The presence or absence of the improvement action of each sample on the mouse contact dermatitis model was evaluated according to the method disclosed above.

### 2. Test result

Table 5 and Fig. 9 show Δ auricle thickness in each group.

**[Table 5]**

| Changed amount of auricle thickness (Δ auricle thickness (mm)) and standard error (SE) | | | | | |
|---|---|---|---|---|---|
| Mouse number | Group A | Group B | Group C | Group D | Group E |
| 1 | 0.16 | 0.18 | 0.10 | 0.16 | 0.16 |
| 2 | 0.18 | 0.16 | 0.12 | 0.16 | 0.18 |
| 3 | 0.16 | 0.16 | 0.12 | 0.10 | 0.14 |
| 4 | 0.16 | 0.14 | 0.10 | 0.16 | 0.12 |
| 5 | 0.14 | 0.18 | 0.10 | 0.14 | 0.12 |
| Average | 0.160 | 0.164 | 0.108 | 0.144 | 0.144 |
| SE | 0.006 | 0.007 | 0.005 | 0.012 | 0.012 |

As can be seen from Table 5 and Fig. 9, it was confirmed that an increase in the auricle thickness due to inflammation was significantly suppressed in the fermented juice administration group (Group C) (p < 0.05), as compared to the positive control group (Group A). In addition, the increase value was lower in Groups D and E, in which the diluted samples of the fermented juice were administered to the mice, than in the control group. In Group B in which the unfermented pineapple juice was administered to the mice, no improvement effect was observed.

The above results showed that the repeated oral administration of the pineapple juice fermented with strain IJH-SONE68 prevented and improved the symptoms of the picryl chloride-induced contact dermatitis model.

### Example 6

### Improvement action of neutral and acidic polysaccharides produced by IJH-SONE68 on mouse contact dermatitis model

Investigations were made on the improvement action of the neutral and acidic polysaccharide produced by the strain IJH-SONE68 on mouse contact dermatitis model, by measuring an auricle thickness change, an inflammatory cytokine IL-4 level, and a serum IgE level, and observing inflammation of auricle, in mouse contact dermatitis model, as disclosed below.

### 1. Measurement of auricle thickness change

### (1) Test method

The mice used were male BALB/cA Jcl mice (7-week old at the time of arrival, CLEA Japan, Inc.). Five mice were housed in a breeding cage and bred in an animal breeding room in which environment was adjusted to temperature of 20 to 26°C, humidity of 40 to 60%, and lighting time of 12 hours (8:00 to 20:00). The mice were allowed to freely ingest solid feed MF (Oriental Yeast Co., Ltd.). In addition, with regard to a drinking water, the mice were allowed to freely ingest a purified water.

After carried in the cage, the mice were acclimated for 7 days. After acclimated breeding, the mice were divided into groups as follows:
Group A: a negative control group (a group in which the mice were bred only with usual diets)
Group B: a positive control group (a sterile distilled water administration group)
Group C: a group in which a solution of a purified acidic EPS (1 mg/mL) was administered to the mice
Group D: a group in which a solution of a purified acidic EPS (0.1 mg/mL) was administered to the mice
Group E: a group in which a solution of a purified neutral EPS (1 mg/mL) was administered to the mice
Group F: a group in which a solution of a purified acidic EPS (0.1 mg/mL) was administered to the mice

Here, the acidic EPS solution of Group C and the neutral EPS solution of Group E were the acidic polysaccharide fractions and the neutral polysaccharide fractions obtained in Example 2, respectively. Those solutions were diluted with a sterile distilled water to prepare the acidic EPS solution of Group D and the neutral EPS solution of Group F.

The time point at which the above grouping was performed was designated as Day 0, and the samples were orally administered to the mice at a frequency of once per day from Day 0 for 14 consecutive days. The daily dose volume was 0.20 mL/mouse. On Day 6, all mice were allowed to memorize an antigen information by applying a 7 w/v% picryl chloride solution on both hands and feet (20 µL), thorax and abdomen (100 µL), and back (100 µL). On Day 13, the thickness of right auricle of the mice was measured in advance with a caliper, and then an allergic reaction was induced by applying 20 µL of a 1 w/v% picryl chloride solution on the front and back of the right auricle. Twenty-four hours later (Day 14), the thickness of the auricle was measured, and the difference before and after the induction (Δ auricle thickness [mm]) was compared among the groups. Data were shown as mean ± standard error. Tukey-Kramer method was used for a significant difference test among the groups, and it was determined that there was a significant difference when a risk factor (p value) was less than 0.05.

The presence or absence of the suppression action of each sample on the mouse contact dermatitis model was evaluated according to the method disclosed above.

### 2. Test result

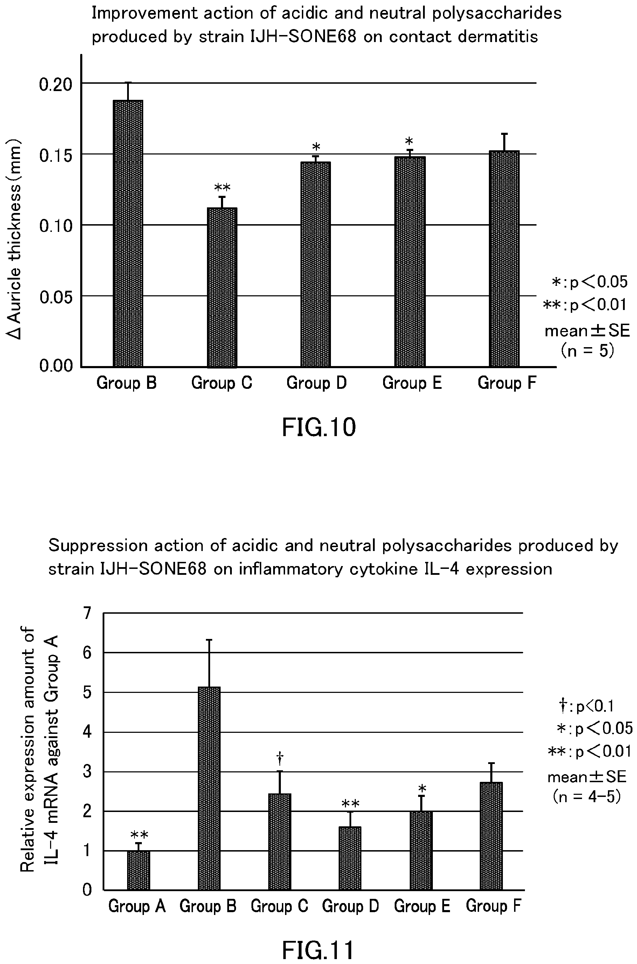

Table 6 and Fig. 10 show Δ auricle thickness in each group.

**[Table 6]**

| Changed amount of auricle thickness (Δ auricle thickness (mm)) and standard error (SE) | | | | | |
|---|---|---|---|---|---|
| Mouse number | Group B | Group C | Group D | Group E | Group F |
| 1 | 0.16 | 0.12 | 0.14 | 0.14 | 0.14 |
| 2 | 0.20 | 0.10 | 0.14 | 0.16 | 0.12 |
| 3 | 0.20 | 0.14 | 0.16 | 0.14 | 0.14 |
| 4 | 0.16 | 0.10 | 0.14 | 0.16 | 0.18 |
| 5 | 0.22 | 0.10 | 0.14 | 0.14 | 0.18 |
| Average | 0.188 | 0.112 | 0.144 | 0.148 | 0.152 |
| SE | 0.012 | 0.008 | 0.004 | 0.005 | 0.012 |

As can be seen from Table 6 and Fig. 10, it was confirmed that an increase in the auricle thickness due to inflammation was significantly suppressed in the acidic EPS solution administration groups both at 1 mg/mL concentration (Group C) and at 0.1 mg/mL concentration (Group D) (p < 0.01 in Group C and p < 0.05 in Group D), as compared to the positive control group (Group B). In addition, in the neutral EPS solution administration group, the significant suppression was observed at 1 mg/mL concentration (Group E, p < 0.05), and an increase in the auricle thickness due to inflammation was also suppressed at 0.1 mg/mL concentration (Group F).

The above results suggested that the repeated oral administration of the acidic or neutral polysaccharide produced by the strain IJH-SONE68 prevented and improved the symptoms of the picryl chloride-induced contact dermatitis model, and it was suggested that there was a possibility that the effect was particularly high in the acidic polysaccharide.

### 2. Measurement of inflammatory cytokine IL-4 level and serum IgE level (1) Test method

After the measurement of the auricle thickness disclosed in the test method in 1 above, the mice were euthanized and, after euthanasia, the right auricle in which inflammation had been caused was excised and collected from the mice of each group, followed by extraction of mRNA with NucleoSpin RNA Plus kit (MACHEREY -NAGEL) cDNA was synthesized from the extracted mRNA sample using ReverTra Ace qPCR RT Master Mix with gDNA Remover kit (TOYOBO). Using the cDNA as a template, the mRNA expression amount of inflammatory cytokine IL-4 at the inflammation induction site was compared by a quantitative RT-PCR method. The quantitative RT-PCR was performed in PikoReal Real-Time PCR System apparatus (manufactured by Thermo Fisher Scientific) using KAPA SYBR FAST qPCR Master Mix (manufactured by Nippon Genetics).

In addition, serum was collected from blood collected after the euthanasia, and serum IgE concentration was measured by an ELISA method using Mouse IgE ELISA development kit (HRP) (manufactured by Mabtech).

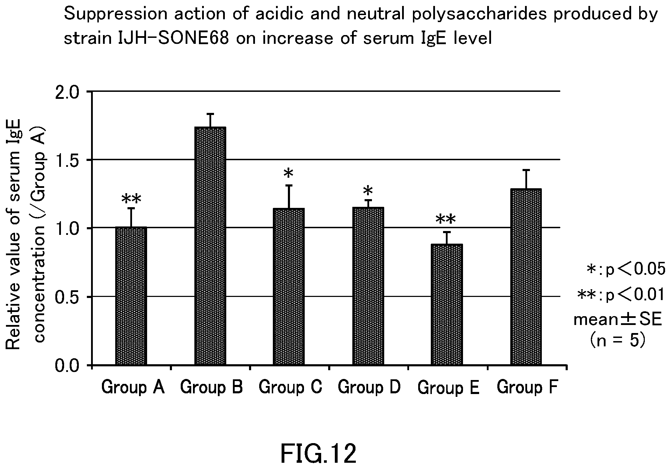

Each data were shown as the mean ± standard error, and Tukey-Kramer method was used for a significant difference test among groups, and it was determined that there was a significant difference when a risk factor (p value) was less than 0.05 (Fig. 11 and Fig. 12 disclosed below show only a significant difference from the positive control group).

### (2) Test results

Table 7 and Fig. 11 show the expression amount of IL-4 mRNA in Groups A to F (the relative expression amount of IL-4 mRNA against Group A).

**[Table 7]**

| Expression amount of IL-4 mRNA and relative expression amount of IL-4 mRNA against Group A | | | | | | |
|---|---|---|---|---|---|---|
| Mouse number | Group A | Group B | Group C | Group D | Group E | Group F |
| 1 | 0.33 | 6.20 | 2.54 | 0.61 | 1.89 | 1.48 |
| 2 | 1.43 | 9.03 | 1.65 | 1.25 | 1.14 | 3.63 |
| 3 | 1.18 | 2.44 | 4.55 | 1.57 | 3.26 | 3.64 |
| 4 | 0.89 | 2.93 | 1.31 | 2.91 | 2.40 | 2.17 |
| 5 | 1.16 | 5.05 | 2.18 | 1.71 | 1.32 | - |
| Average | 1.00 | 5.13 | 2.44 | 1.61 | 2.00 | 2.73 |
| SE | 0.19 | 1.19 | 0.57 | 0.38 | 0.38 | 0.48 |

As can be seen from Table 7 and Fig 11, the transcription of the mRNA of inflammatory cytokine IL-4 was significantly increased due to the inflammation induction in the positive control group (Group B) (p < 0.01). In the groups in which the mice ingested each polysaccharide, a significant decrease (p < 0.01 in Group D and p < 0.05 in Group E) or a decreasing tendency (p < 0.1 in Group C) was observed as compared to the positive control group (Group B).

Table 8 shows serum IgE concentration (ng/mL) in Groups A to F, and Table 9 shows the relative value of serum IgE concentration in Groups A to F against Group A. The relative values are graphed in Fig. 12.

**[Table 8]**

| Serum IgE concentration (ng/mL) | | | | | | |
|---|---|---|---|---|---|---|
| Mouse number | Group A | Group B | Group C | Group D | Group E | Group F |
| 1 | 210.2 | 627.0 | 477.0 | 375.7 | 376.8 | 424.2 |
| 2 | 349.4 | 485.3 | 361.2 | 388.2 | 189.2 | 486.2 |
| 3 | 461.1 | 596.3 | 334.5 | 331.0 | 331.2 | 408.7 |
| 4 | 396.8 | 518.1 | 528.1 | 397.3 | 283.2 | 270.6 |
| 5 | 258.9 | 657.7 | 206.7 | 429.5 | 287.1 | 549.2 |
| Average | 335.3 | 576.9 | 381.5 | 384.3 | 293.5 | 427.8 |
| SE | 45.43 | 32.61 | 56.48 | 16.04 | 31.13 | 46.49 |

**[Table 9]**

| Relative value of serum IgE concentration against Group A (/Group A) | | | | | | |
|---|---|---|---|---|---|---|
| Mouse number | Group A | Group B | Group C | Group D | Group E | Group F |
| Average | 1.00 | 1.72 | 1.14 | 1.15 | 0.88 | 1.28 |
| SE | 0.14 | 0.10 | 0.17 | 0.05 | 0.09 | 0.14 |

As can be seen from Tables 8 and 9 and Fig. 12, the serum IgE concentration was significantly increased in the positive control group (Group B), like the case of IL-4. A significant decrease was observed in the groups in which the mice ingested each polysaccharide (particularly Groups C to E), as compared to the positive control group.

The above results show that the repeated oral administration of the acidic and neutral polysaccharides produced by the strain IJH-SONE68 suppressed the expression level of inflammatory cytokine IL-4 and serum IgE level in the picryl chloride-induced contact dermatitis model mice.

### 3. Observation of auricle inflammation

### (1) Test method

After the measurement of the auricle thickness disclosed in the test method in 1 above, the mice were euthanized and, after euthanasia, the right auricle in which inflammation had been caused was excised and collected from the mice of Groups A to F. The collected auricle was fixed with a 10% formalin solution, and then embedded in paraffin to prepare a tissue section. The sections were stained with hematoxylin-eosin (HE) and the state of inflammation was observed under microscope.

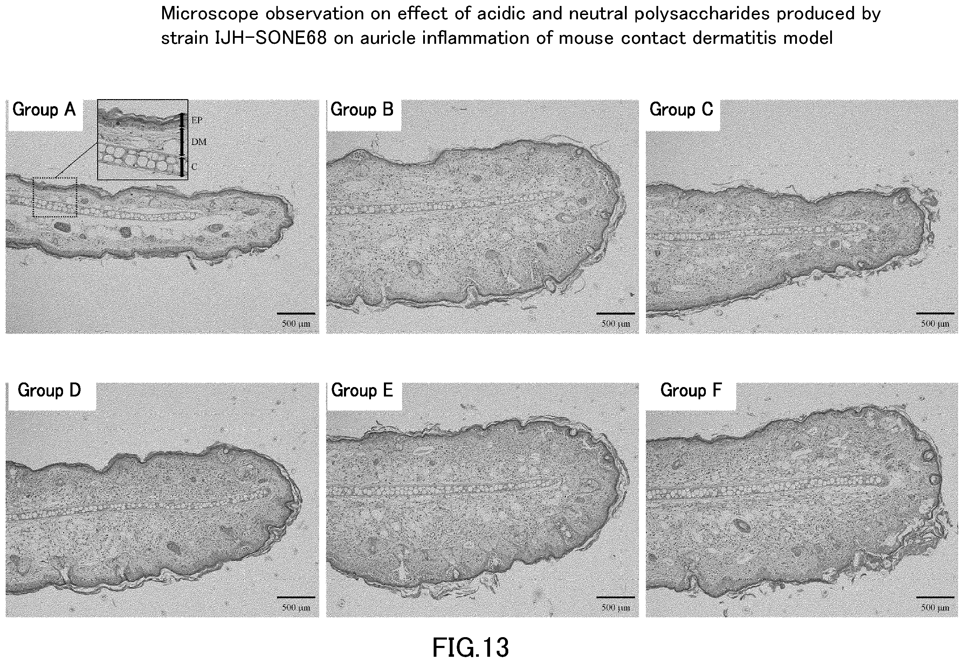

Fig. 13 shows the results. As can be seen from Fig. 13, while epidermal layer (EP) was thin in the positive control (Group B) as compared to the negative control (Group A), the thickness was observed to be clearly increased in dermal layer (DM) due to inflammation. The inflammatory symptoms appeared to be suppressed in proportion to the reduction in auricle thickness with the administration of each polysaccharide sample.

### Industrial Applicability

As disclosed in detail herein above, the composition of the present invention is effective in preventing or improving type IV allergy and can be used to prevent or improve particularly contact dermatitis. The composition of the present invention can be used as a food or drink, a medicine, a feed, and a cosmetic product, for the prevention or improvement of type IV allergy including contact dermatitis. The composition of the present invention comprises, as an active ingredient, a cell body of a lactic acid bacterium belonging to *Lactobacillus paracasei,* a cultured product or fermented product thereof, or a polysaccharide produced thereby, as defined in the appended claims. Therefore, the composition of the present invention has high safety, can be applied for a long period of time, can be supplied in a large amount at low cost, and has extremely high utility and practicality.

## Claims

1. A composition for use in the prevention or improvement of type IV allergy which is contact dermatitis, the composition comprising, as an active ingredient, a cell body of a lactic acid bacterium belonging to *Lactobacillus paracasei,* a cultured product or fermented product thereof, or a polysaccharide produced thereby, wherein the lactic acid bacterium is *Lactobacillus paracasei* strain IJH-SONE68 (Accession No. NITE BP-02242), and wherein the polysaccharide is a neutral polysaccharide having a structure in which N-acetylglucosamines are linked with each other via α-1,6 bond and/or an acidic polysaccharide composed mainly of glucoses and mannoses.

2. The composition for use according to claim 1, wherein the cultured product or fermented product of the lactic acid bacterium is a cultured product or fermented product that is obtained by cultivating or fermenting the lactic acid bacteria in the presence of a fruit juice of pineapple genus plant.

3. The composition for use according to any one of claims 1 to 2, wherein the composition is a food or drink composition.

4. The composition for use according to claim 3, wherein the food or drink is a beverage, a functional food, a fermented food, or a supplement.

5. The composition for use according to any one of claims 1 to 2, wherein the composition is a pharmaceutical composition.

6. The composition for use according to any one of claims 1 to 2, wherein the composition is a feed composition.

7. The composition for use according to any one of claims 1 to 2, wherein the composition is a cosmetic composition.

## Patentansprüche

1. Zusammensetzung zur Verwendung bei der Vorbeugung oder Verbesserung von Typ-IV-Allergie, die Kontaktdermatitis ist, wobei die Zusammensetzung als einen Wirkstoff einen Zellkörper eines Milchsäurebakteriums, das zu ***Lactobacillus paracasei,*** einem kultivierten Produkt oder einem fermentierten Produkt davon, oder ein dadurch hergestelltes Polysaccharid aufweist, wobei das Milchsäurebakterium ***Lactobacillus paracasei***-Stamm IJH-SONE68 (Hinterlegungsnummer NITE BP-02242) ist, und wobei das Polysaccharid ein neutrales Polysaccharid mit einer Struktur, in der N-Acetylglucosamine über eine α-1,6-Bindung miteinander verbunden sind, und/oder ein saures Polysaccharid ist, das sich hauptsächlich aus Glucosen und Mannosen zusammensetzt.

2. Zusammensetzung zur Verwendung gemäß Anspruch 1, wobei das kultivierte Produkt oder fermentierte Produkt des Milchsäurebakteriums ein kultiviertes Produkt oder fermentiertes Produkt ist, das durch Kultivieren oder Fermentieren der Milchsäurebakterien in Gegenwart eines Fruchtsafts einer Pflanze der Gattung Ananas erhalten wird.

3. Zusammensetzung zur Verwendung nach einem der Ansprüche 1 bis 2, wobei die Zusammensetzung eine Lebensmittel- oder Getränkezusammensetzung ist.

4. Zusammensetzung zur Verwendung nach Anspruch 3, wobei das Lebensmittel oder Getränk ein Getränk, ein funktionelles Lebensmittel, ein fermentiertes Lebensmittel oder ein Ergänzungsmittel ist.

5. Zusammensetzung zur Verwendung nach einem der Ansprüche 1 bis 2, wobei die Zusammensetzung eine pharmazeutische Zusammensetzung ist.

6. Zusammensetzung zur Verwendung nach einem der Ansprüche 1 bis 2, wobei die Zusammensetzung eine Futterzusammensetzung ist.

7. Zusammensetzung zur Verwendung nach einem der Ansprüche 1 bis 2, wobei die Zusammensetzung eine Kosmetikzusammensetzung ist.

## Revendications

1. Composition pour une utilisation dans la prévention ou l'amélioration d'une allergie de type IV qui est une dermite de contact, la composition comprenant, en tant que principe actif, un corps cellulaire d'un ferment lactique appartenant à *Lactobacillus paracasei,* d'un produit cultivé ou d'un produit fermenté de celui-ci, ou d'un polysaccharide produit par celui-ci, dans laquelle le ferment lactique est la souche IJH-SONE68 de *Lactobacillus paracasei* (numéro d'accès NITE BP-02242), et dans laquelle le polysaccharide est un polysaccharide neutre ayant une structure dans laquelle des N-acétylglucosamines sont liées entre elles via une liaison α-1,6 et/ou un polysaccharide acide composé principalement de glucoses et de mannoses.

2. Composition pour une utilisation selon la revendication 1, dans laquelle le produit cultivé ou le produit fermenté du ferment lactique est un produit cultivé ou un produit fermenté qui est obtenu par culture ou fermentation du ferment lactique en présence d'un jus de fruit d'une plante du genre ananas.

3. Composition pour une utilisation selon l'une quelconque des revendications 1 et 2, dans laquelle la composition est une composition de type aliment ou boisson.

4. Composition pour une utilisation selon la revendication 3, dans laquelle l'aliment ou la boisson est une boisson, un aliment fonctionnel, un aliment fermenté, ou un complément.

5. Composition pour une utilisation selon l'une quelconque des revendications 1 et 2, dans laquelle la composition est une composition pharmaceutique.

6. Composition pour une utilisation selon l'une quelconque des revendications 1 et 2, dans laquelle la composition est une composition alimentaire pour animaux.

7. Composition pour une utilisation selon l'une quelconque des revendications 1 et 2, dans laquelle la composition est une composition cosmétique.
